# EUROPEAN PATENT SPECIFICATION

(11) **EP 2 387 411 B1**
(45) Date of publication and mention of the grant of the patent: **06.03.2019**
(21) Application number: 10702926.6
(22) Date of filing: 15.01.2010
(51) Int. Cl.: A61K 36/88, A61P 1/00

(54) **GIP-INCREASE INHIBITOR**
GIP-ZUNAHME INHIBITOR
INHIBITEUR D'AUGMENTATION DE GIP

(30) Priority: 16.01.2009 JP 2009007798; 01.12.2009 JP 2009273500
(43) Date of publication of application: 23.11.2011
(73) Proprietor: Kao Corporation, Chuo-Ku Tokyo 103-8210 (JP)
(72) Inventor: FUKUOKA, Daisuke, Haga-gun Tochigi 321-3497 (JP); HASHIZUME, Kohjiro, Haga-gun Tochigi 321-3497 (JP); SHIMOTOYODOME, Akira, Haga-gun Tochigi 321-3497 (JP)
(74) Representative: Vossius & Partner Patentanwälte Rechtsanwälte mbB
(86) International application number: PCT/JP2010/050750
(87) International publication number: WO 2010/082689

(56) References cited:
- DATABASE WPI Week 200343 Thomson Scientific, London, GB; AN 2003-451481 XP002576109 & JP 2002 316939 A (REAL KK) 31 October 2002 (2002-10-31)
- DATABASE WPI Week 199946 Thomson Scientific, London, GB; AN 1999-543862 XP002576110 & JP 11 225717 A (KAJIWARA M) 24 August 1999 (1999-08-24)
- CICERO A F G ET AL: "Rice bran and its main components: Potential role in the management of coronary risk factors" CURRENT TOPICS IN NUTRACEUTICAL RESEARCH, NEW CENTURY HEALTH PUBLISHERS, NEW ORLEANS, LA, US, vol. 3, no. 1, 1 January 2005 (2005-01-01) , pages 29-46, XP009131719 ISSN: 1540-7535
- CHIA-WEN CHEN, HSING-HSIEN CHENG: "A rice bran oil diet increases LDL-Receptor and HMG-CoA Reductase mRNA Expressions and Insulin Sensitivity in rats with Streptozotocin/Nicotinamide-induced type 2 Diabetes" JOURNAL OF NUTRITION, vol. 136, no. 6, June 2006 (2006-06), pages 1472-1476, XP009131706 ISSN: 0022-3166
- YASUKAWA K ET AL: "Inhibitory effect of cycloartenol ferulate, a component of rice bran, on tumor promotion in two-stage carcinogenesis in mouse skin" BIOLOGICAL & PHARMACEUTICAL BULLETIN (OF JAPAN), PHARMACEUTICAL SOCIETY OF JAPAN, TOKYO, JP, vol. 21, no. 10, 1 January 1998 (1998-01-01), pages 1072-1076, XP003020948 ISSN: 0918-6158
- ROBERT R.WILLIAMS: "The world beriberi problem today" J. CLIN. NUTRIT. 1953, vol. 1, no. 7, 1953, pages 513-516, XP009131709
- JIANG YONGZHI ET AL: "Phytosterols in cereal by-products" JOURNAL OF THE AMERICAN OIL CHEMISTS' SOCIETY, SPRINGER, BERLIN, DE, vol. 82, no. 6, 1 June 2005 (2005-06-01), pages 439-444, XP009131717 ISSN: 0003-021X

## Description

### Field of the Invention

The present application discloses to a novel GIP-increase inhibitor. The present invention relates to a non-therapeutic in vivo use of a rice bran extract for inhibiting GIP-increase.

### Background of the Invention

Gastric inhibitory polypeptide (GIP) is a gastrointestinal hormone exhibiting a gastric acid secretion inhibitory action or gastric motility inhibitory action, and it is known that the secretion is enhanced by lipids and the like contained in the diet during food intake (Non-Patent Documents 1 to 3). Therefore, a substance inhibiting the secretion of GIP is believed to be useful for facilitation of digestion or improvement of a heavy feeling in the stomach. Previous studies have reported that 3-bromo-5-methyl-2-phenylpyrazolo[1,5-a]pyrimidin-7-ol (BMPP) is a substance which inhibits the functions of GIP, and that guar gum and the like are substances which inhibit the secretion of GIP (Patent Document 1 and Non-Patent Documents 4 to 9) .

However, there are problems in that the former substance has not been verified to have an inhibitory effect on the functions of GIP in vivo, and the GIP secretion inhibitory effect during lipid ingestion of the latter substances has not been examined. Further, the effect of the above substances for improving a heavy feeling in the stomach or the like is not always satisfactory, and thus a substance which is safe to ingest on a daily basis and exhibit more excellent effect is desired.

Meanwhile, rice bran refers to a portion other than an endosperm, the portion being removed when brown rice is polished to white rice, and contains many kinds of nutrients such as dietary fibers (cellulose), proteins, lipids, vitamins, and minerals, and functional ingredients such as γ-orizanol, phytic acid, and free γ-aminobutyric acid. It has been reported until now that a rice bran extract has an adiponectin secretion accelerating action, an anti-cholesterol action, an anti-cancer action, and an action for improving an alcoholic liver disease (Patent Document 2 and Non-patent Documents 10 to 12).

However, there has been no report regarding relationship between rice bran extract and GIP, and in particular, relationship between rice bran oil and GIP.

### Prior Art Documents

### Patent Documents

Patent Document 1: WO 01/87341 pamphlet
Patent Document 2: JP-A-2005-68132

### Non-Patent Documents

Non-Patent Document 1: J. C. Brown, et al., Canadian J. Physiol. Pharmacol., 47: 113-114, 1969
Non-Patent Document 2: J.M. Falko, et al., J. Clin. Endocrinol. Metab., 41 (2): 260-265, 1975
Non-Patent Document 3: Oda Toshitsugu, et al., Digestive Tract-Functions and Pathological conditions ("Shoukakan Kinou to Byoutai"), 1981, Chugai-Igakusha, pp. 201-218
Non-Patent Document 4 : Gagenby S J, et al., Diabet. Med., 1996 April; 13 (4): 358-64
Non-Patent Document 5: Ellis P R, et al., Br. J. Nutr., 1995 October; 74 (4): 539-56
Non-Patent Document 6: Simoes Nunes C, et al., Reprod. Nutr. Dev., 1992; 32 (1): 11-20
Non-Patent Document 7 : Morgan L M, et al., Br. J. Nutr. , 1990 July; 64 (1): 103-10
Non-Patent Document 8: Requejo F, et al., Diabet. Med., 1990 July; 7 (6): 515-20
Non-Patent Document 9: Morgan, et al. , Br. J. Nutr. , 1985 May; 53 (3): 467-75
Non-Patent Document 10: Chen, et al., J. Nutr., 2006 June; 136 (6): 1472-6
Non-Patent Document 11: Ullah, et al., Carcinogenesis., 1990 December; 11 (12): 2219-22
Non-Patent Document 12: Oh, et al., J. Med. Food., 2003 Summer; 6 (2): 115-21

### Summary of the Invention

The present application discloses a GIP-increase inhibitor, containing a rice bran extract as an active ingredient.

The present application also discloses a medical composition for inhibiting GIP-increase, containing a rice bran extract and a pharmaceutically acceptable carrier.

The present application also discloses a method of inhibiting GIP-increase, containing administration or ingestion of a rice bran extract.

The present invention also provides a non-therapeutic in vivo use of a rice bran extract for inhibiting GIP-increase.

The present application also discloses a rice bran extract for facilitation of digestion and/or improvement of a heavy feeling in a stomach after eating.

### Brief Description of the Drawing

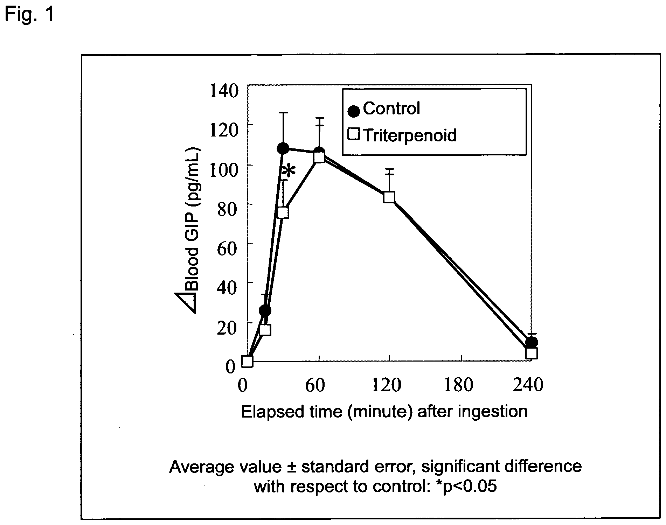

FIG. 1 is a graph illustrating a time-dependent change of GIP level in blood after ingestion of cooked rice.

### Detailed Description of the Invention

The present invention provides a GIP-increase inhibitor, which is useful as a food. The present application discloses a GIP-increase inhibitor, which is useful as a drug.

The inventors of the present invention have studied materials capable of controlling GIP-increase, and have found out that a rice bran extract remarkably inhibits the GIP-increase, and is useful for facilitation of digestion and an improvement of a heavy feeling in the stomach.

The present invention may contribute to inhibition of the GIP-increase, to thereby facilitate digestion after eating and improve a heavy feeling in the stomach.

The terms "GIP-increase inhibition" used herein refer to inhibition of the increase of a blood level of GIP secreted from the gastrointestinal tract by ingestion of a lipid and/or carbohydrate containing diet, in particular, a high lipid containing diet, or in more particular, a high triacylglycerol containing diet. That is, the terms "GIP-increase inhibition" refer to inhibition of GIP-increase mainly occurring after eating. Further, the terms "GIP-increase inhibitory action" in the present invention refer to a concept that includes both a GIP secretion inhibitory action by which GIP-increase is inhibited by inhibition of GIP secretion from the gastrointestinal tract, and a GIP decrease action by which GIP-increase is inhibited by decreasing a blood GIP level.

In addition, the term "non-therapeutic" in the present invention refers to a concept that does not include medical practice (i.e., medical treatment of a human body or an animal body through therapy).

Brown rice is formed of an episperm, a pericarp, an endosperm, and a germ, and the outer layer of endosperm tissue is an aleurone layer. Whenbrownrice is subjectedtograinpolishing, the episperm, the pericarp, the germ, and the aleurone layer are eliminated as rice bran (fresh rice bran) depending on the degree of the grain polishing.

The rice bran to be used in the present invention may be either a mixture of portions which are other than an endosperm and are removed when brown rice is subjected to grain polishing (an episperm, a pericarp, an aleurone layer, and an germ), or a product obtained by isolating any of them. The species or kinds of rice serving as a material for rice bran are not particularly limited, and any kind of rice, such as glutinous rice, non-glutinous rice, red rice, and purple rice of japonica variety, indica variety, and javanica variety, may be used.

The rice bran extract used in the present invention includes an extract obtained by appropriate grounding and processing of rice bran (with or without further drying or freeze-drying process of the rice bran beforehand) and subj ecting the resultant to extraction, and further includes a more active fraction (ingredient) obtained by subjecting the resultant extract to separation and purification. Specific examples thereof include a rice bran oil, and an ingredient concentrated oil obtained by subjecting the rice bran oil to an enzymatic treatment, followed by distillation and deacidification.

Commercially available extracts of rice bran may also be used. There may be used a rice bran extract obtained, for example, by production method described in JP 2006-A-257064, the method comprising dissolving rice bran in ethanol under heating (80°C for 1 hour) followed by concentration, adding hexane to the resultant to dissolve the concentrate, further adding sulfuric acid to adjust the pH of the mixture to 3 followed by filtration, extracting the filtrate with ethanol and sodium hydroxide while refluxing (80°C for 1 hour), leaving the resultant to stand still to obtain a supernatant, adding hydrochloric acid to the supernatant for neutralization, and subjecting the resultant to concentration, drying, and solidification.

Extraction may be carried out by following solvent extraction method using performing immersion in a solvent at room temperature or at high temperature or using an extractor such as a Soxhlet extractor, as well as an extraction method using a distillation method such as steam distillation, a supercritical extraction method using carbon dioxide in a supercritical state, and a squeezing method involving squeezing, to provide an extract. The extraction is preferred to be carried out using 1 to 50 parts by weight of a solvent with respect to 1 part by weight of rice bran and subjecting the mixture to immersion or reflux under heating at room temperature (25°C) to 100°C for several hours to several weeks.

For example, the rice bran oil is a vegetable oil obtained from rice bran and rice germ, as a material, by extraction, and is obtained by, for example, an extraction method containing compressing rice bran to yieldpellets, then extracting an oil content therefrom, and subjecting the resultant to a distillation treatment for purification. Specifically, 10 parts by weight of a freeze-dried product of rice bran are added to 100 parts by weight of a solvent, the mixture is subjected to extraction under stirring for several hours, the extract is filtrated to remove solid contents, and the resultant is then purified by distillation. A method of subjecting the resulting rice bran oil to an enzymatic treatment includes, for example, a method of treating the rice bran oil at 50°C for 3 to 20 hours in the presence of lipase. Further, a method of subjecting the resulting rice bran oil to distillation and deacidification includes, for example, a method containing using a Kugel distillation apparatus under the conditions of 220°C and 13.3 Pa.

An extraction solvent used for solvent extraction includes, for example, alcohols such as methanol, ethanol, propanol, and butanol; polyols such as ethylene glycol, propylene glycol, and butylene glycol; ketones such as acetone and methyl ethyl ketone; esters such as methyl acetate and ethyl acetate; linear or cyclic ethers such as tetrahydrofuran and diethyl ether; polyethers such as polyethylene glycol; halogenated hydrocarbons such as dichloromethane, chloroform, and carbon tetrachloride; hydrocarbons such as hexane, cyclohexane, and petroleum ether; aromatic hydrocarbons such as benzene and toluene; pyridines; supercritical carbon dioxide; and fats and oils, wax, and other oils. Those solvents may be used alone or in a combination of two or more kinds, and the solvent extraction may be repeated by changing the solvent. Of those solvents, lipophilic solvents such as hydrocarbons are preferably used, and hexane is more preferably used.

Further, a method of subjecting an extract to separation and purification includes, for example, subjecting the extract to an activated carbon treatment, liquid-liquid distribution, column chromatography, liquid chromatography, gel filtration, and precision distillation.

As a rice bran extract used in the present invention, the extract liquid or its fraction thus obtained may be used directly, a diluted liquid obtained by diluting the rice bran extract with a suitable solvent may be used, or a product prepared by making the rice bran extract into a form of a concentrated extract, a dried powder or paste may be used. In addition, the rice bran extract used in the present invention may be freeze-dried and be used after diluting the freeze-dried product with a solvent usually used for extraction just before the use. Further, the rice bran extract used in the present invention may also be used by being contained in vesicle such as liposome, a microcapsule or the like.

The rice bran extract used in the present invention preferably contains triterpenes or derivatives thereof. Examples of the triterpenes or derivatives thereof include lupane-type triterpenes or derivatives thereof, cucurbitane-type triterpenes or derivatives thereof, and cycloartane-type triterpenes or derivatives thereof. Of those, preferred are cycloartane-type triterpenes or derivatives thereof such as cycloartenol and 24-methylenecycloartanol, and particularly preferred are cycloartenol or derivatives thereof.

As those derivatives, esters such as fatty acid esters, ferulic acid esters, and cinnamic acid esters and glycosides such as saponin are exemplified.

The rice bran extract used in the present invention contains triterpenes or derivatives thereof preferably 0.01 weight% or more, particularly preferably 0.1 to 100 weight%, more preferably 1 to 100 weight%, or even more preferably 10 to 100 weight%. In addition, the rice bran extract used in the present invention contains cycloartane-type triterpenes or derivatives thereof preferably 0.01 weight% or more, particularly preferably 0.1 to 100 weight%, more preferably 1 to 100 weight%, or even more preferably 10 to 100 weight%. Further, the rice bran extract used in the present invention contains cycloartenol or derivatives thereof preferably 0.01 weight% or more, particularly preferably 0.1 to 100 weight%, more preferably 1 to 100 weight%, or even more preferably 10 to 100 weight%.

As shown in the examples described below, the rice bran extract exhibits an action of significantly inhibiting GIP-increase after eating. Thus, the rice bran extract may be used as a GIP-increase inhibitor, or may be used, based on its ability of inhibiting GIP-increase, as an agent for facilitating digestion, an agent for improving a heavy feeling in the stomach, an agent for improving a gastric acid secretion ability, or the like (hereinafter, referred to as "GIP-increase inhibitor or the like"), and may be further used for formulation of those agents. In this case, in the GIP-increase inhibitor or the like, there may be used the rice bran extract alone, or in addition to it, there may be used acceptable substances such as a carrier being selected appropriately as needed, which is acceptable for substances for mixing with the extract described below. It should be noted that the formulation may be produced by an ordinary method depending on the object substances that have to be mixed.

The GIP-increase inhibitor or the like maybe used for adding, as an active ingredient, into a drug for a human being or an animal, a quasi drug, a food, or a feed, wherein the active ingredient exerts, for example, an effect of inhibiting GIP-increase, an effect of facilitating digestion after eating, and an effect of improving a heavy feeling in the stomach, and an effect of improving a gastric acid secretion ability. Further, the GIP-increase inhibitor or the like is applicable, as needed, to a food, a functional food, a hospital food, a food for specified health use or the like, which are prepared for facilitation of digestion after eating, improvement of a heavy feeling in the stomach, and improvement of a gastric acid secretion ability, and in which the above-mentioned effects are indicated.

When the GIP-increase inhibitor or the like disclosed in the present application is used as an active ingredient in a drug, the drug may be administered in any administration form. Examples of the administration form include oral administration of a tablet, a capsule, a granule, a powder, a syrup, or the like, or parenteral administration of an injection, a suppository, an inhalant, a transdermal drug, an external drug, or the like.

In order to prepare those pharmaceutical preparations in various formulations, the GIP-increase inhibitor or the like disclosed in the present application may be used alone, or may be used in appropriate combination with other pharmaceutically acceptable excipient, binder, expander, disintegrant, surfactant, lubricant, dispersant, buffer, preservative, savoring agent, fragrance, coating, carrier, diluent, and the like.

Of these administration forms, oral administration is preferred. The content of the rice bran extract in a preparation for oral administration, containing the GIP-increase inhibitor or the like, is generally 0.01 to 100 weight%, preferably 0.1 to 100 weight%, or more preferably 1 to 100 weight%.

When the GIP-increase inhibitor or the like disclosed in the present application is used as an active ingredient in a food, examples of the form of the food include various foods such as breads, cakes, noodles, confectioneries, jellies, frozen foods, ice creams, dairy products and beverages, as well as the same forms as the above-mentioned forms of the preparations for oral administration (a tablet, a capsule, a syrup, or the like).

In order to prepare those foods in various forms, the GIP-increase inhibitor or the like disclosed in the present application may be used alone, or may be used in appropriate combination with other food materials, a solvent, a softener, oil, an emulsifier, antiseptic, fragrance, a stabilizer, a colorant, an antioxidant, a humectant, thickener, and the like. The content of the rice bran extract in those foods is, in general, preferably 0.01 to 100 weight%, more preferably 0.1 to 100 weight%, or even more preferably 1 to 100 weight%.

The administration amount or ingestion amount of the above-mentioned preparations may vary depending on the condition, body weight, sex, and age of a patient or other factors, the amount for oral administration or ingestion per adult per day is, in terms of cycloartenol, preferably 50 µg to 500 mg, particularly preferably 75 µg to 100 mg, more preferably 100 µg to 20 mg, or even more preferably 150 µg to 5 mg.

Further, the GIP-increase inhibitor or the like disclosed in the present application is administered or ingested preferably during or before food intake, particularly preferably 5 minutes to 30 minutes before the food intake or feed intake. Subjects for administration or ingestion are preferably patients whose fasting blood GIP value is 30 pg/mL or more, or patients whose basic amount of gastric secretion is 30 mL/hour or less in the gastric secretion function test.

### EXAMPLE

### Example 1

### (Preparation of rice bran extract)

(1) 1.8 kg of rice bran prepared from Koshihikari, produced in Tochigi prefecture, were freeze-dried, and 9 L of n-hexane were added to the freeze-dried product, followed by stirring for 3 hours to perform extraction. The extract was filtrated, and the residue was further subjected to extraction twice with 9 L each of n-hexane. Extracts obtained from total three extractions were combined and the resultant was subjected to concentration and drying to yield 372 g of rice bran extract.
(2) To 20 g of the rice bran extract obtained in the above process (1), 15 mL of water and 20 mg of lipase OF (manufactured by Meito Sangyo Co., Ltd.) were added, and the mixture was stirred at 50°C for 15 hours. Then, aqueous layer was removed following centrifugation, and oil layer was subjected to distillation and deacidification under conditions of 220°C and 13.3 Pa to yield 3.2 g of ingredient enriched oil.
(3) A rice bran extract ("ORYZA TRITERPENOID-P" (manufactured by Oryza Oil & Fat Chemical Co., Ltd.)) was subjected to separation and purification. As a result, purified cycloartenol and purified 24-methylene-cycloartanol were obtained. It should be noted that the rice bran extract was produced by powdering of triterpenoids obtained from hydrolysis of rice bran and rice germ derived from *Oryza sativa Linne* seeds. Table 1 shows the results of composition analysis of this product by capillary gas chromatography.

**[Table 1]**

| LOT NO. | Campesterol | β-sitosterol | cycloartenol | 24-methylenecycloartanol |
|---|---|---|---|---|
| (%) | 16^{a)} | 13^{a)} | 19^{a)} | 33^{b)} |

| | | | | |
|---|---|---|---|---|
| a) absolute quantitative value (w/w%) determined based on the calibration curve prepared using a standard cycloartenol b) absolute quantitative value (w/w%) determined based on the calibration curve prepared using a standard 24-methylene-cycloartanol | | | | |

### (Analysis conditions)

Column: capillary GC column DB-1 (manufactured by Agilent Technologies, Inc.), 30 m×0.25 mm, thickness 0.25 µm
Carrier gas: helium, 2.30 mL/min
Split ratio: 40:1
Injector: T=300°C
Detector: FID, T=300°C
Oven temperature: maintain for 1.5 minutes at 150°C, raise temperature at a rate of 15°C/min up to 250°C, raise temperature at a rate of 5°C/min up to 320°C, and maintain for 20 minutes

Specifically, 5 g of "ORYZA TRITERPENOID-P" were fractionated by silica gel column chromatography (developing solvent: hexane/ethyl acetate=9/1) to yield 3.93 g of a fraction containing cycloartenol and 24-methylene-cycloartanol. Then, 1.4 g of the fraction were fractionated by HPLC (developing solvent: methanol/acetonitrile/tetrahydrofuran/water=15/2/2/1) using ODS column (Inertsil ODS-3 : GL Sciences) . As a result, 480 mg of purified cycloartenol and 764 mg of purified 24-methylene-cycloartanol were obtained.

### Test Example 1 (GIP-increase inhibitory action of rice bran extract)

### (1) Preparation of sample

The above-mentioned "ORYZA TRITERPENOID-P" (manufactured by Oryza Oil & Fat Chemical Co., Ltd.) was used as a rice bran extract. These ingredients shown in Table 2 were diluted with potable water by 10 folds to prepare an emulsified liquid. To commercially available white rice (the amount being equivalent to 50 g of carbohydrates), the emulsified liquid (the amount being 1.5 times equivalent to the weight of the white rice) was added to allow the rice to absorb the liquid for 30 minutes. Then, sample (i) and (ii) were prepared by cooking with a rice cooker (ZOJIRUSHI microcomputer-controlled rice cooker NS-KG05, manufactured by Zojirushi Corporation).
(i) Cooked rice (control): about 153 g (50 g of carbohydrates)
(ii) Cooked rice (rice bran extract is added): about 153 g (50 g of carbohydrates and 13 mg of rice bran triterpene)

**[Table 2]**

| Common name | Trade name | Manufacturer's name of material | Blending ratio (wt%) | |
|---|---|---|---|---|
| | | | (i) Cooked rice (control group) | (ii) Cooked rice (rice bran extract is added) (rice bran extract ingestion group) |
| Rice bran extract | ORYZA TRITERPEN OID-P | Oryza Oil & Fat Chemical Co., Ltd. | 0.00 | 0.24 |
| Fat and oil | 73DAG | Kao Corporation | 2.13 | 2.13 |
| Emulsifier | A181E | Taiyo Kagaku Co., Ltd. | 0.44 | 0.44 |
| Potable water | - | - | 97.43 | 97.19 |

### (2) Test method

Five normal male subjects (from 27 to 46 years old) were selected for the test. Drinking alcohol was forbidden from the day before the measurement day, predetermined meals were taken between 7 and 8 o' clock in the evening of the same day, and afterward only potable water was permitted to be drunk until 7 o'clock in the morning of the test day. Meanwhile, they were not allowed to do hard exercise involving sweating.

Five healthy male subjects came to the test place on the test day without taking any breakfast. Around 9 o' clock in the morning, they ingested the whole test product by 30 times chewing per one mouthful, and their blood sample was taken sequentially. The blood sampling was performed totally 6 times, that is, before the ingestion of the test product (0), and each of 15 minutes, 30 minutes, 1 hour, 2 hours, and 4 hours after the ingestion of the test product. 5 mL of blood sample was collected at each time. To the blood sample, immediately a DPPIV inhibitor (manufactured by Linco Research Inc., "LINCO's DPPIV inhibitor", 50 µL for each 5 mL blood) and a serine protease inhibitor (manufactured by Roche Diagnostics GmbH, "Pefabloc SC", 50 µL for each 5 mL blood) were added. The resultant was stored on ice and then within 30 minutes after blood sampling subjected to centrifugation, to prepare blood plasma, which was stored at -80°C until the measurement day.

Drinking water was forbidden until 2 hours after the ingestion of the test product, and afterward drinking only predetermined potable water was allowed until the termination of the test (4 hours after the ingestion of the test product).

Second ingestion test was carried out with 6 day or more interval from the first ingestion test and the male subjects were exchanged with the other test product. GIP in the blood plasma was measured using a "HUMAN GUT HORMONE LINCOplex KIT" (manufactured by Linco Research Inc.).

### (3) Test results

FIG. 1 shows the results of the test. Each point is represented as average ± standard error, and statistical significant differences between groups were determined by the t-test with respect to the control group (*p<0.05).

As shown in FIG. 1, blood GIP level significantly dropped 30 minutes after the eating in the rice bran extract ingestion group compared to the control group, showing that the rice bran extract exhibits a GIP-increase inhibitory effect.

### Test Example 2 (GIP-increase inhibitory action of cycloartenol during lipid stress)

### (1) Preparation of sample and test method

Triolein (manufactured by Sigma-Aldrich Corporation) was added to water at 5% concentration, and the mixture was emulsified with ultrasonication until it was sufficiently homogenized, thereby yielding a sample for administration. The sample for administration was orally probe-administered at 2 mg of triolein per g body weight (as a control group). Each of the purified cycloartenol produced in the above-mentioned Example 1 (3) was added to a sample for administration (dissolved in triolein) . Each of the resultants was orally probe-administered at 2 mg of triolein per g body weight and 0.15 µg or 2 µg of cycloartenol equivalent per g body weight (as Test group 1 or Test group 2).

8-week old male mice, C57BL/6J (supplied by CLEA Japan, Inc.) after 15 hours of fasting were divided into groups each consisting of 10 mice, with their average body weight being almost the same between the groups. Immediately after the administration to each group, blood sample was collected from orbital sinus sequentially. The blood sample was subjected to centrifugation to yield blood plasma, and GIP level in the blood plasma was measured using a GIP measuring kit (manufactured by Linko Research Inc.), and the area under the curve (AUC) in the graph was calculated.

### (2) Test results

Table 3 shows the results of the test. The results are represented as average ± standard error, and statistical significant differences between groups were determined by the Dunnett's test with respect to the control group (*p<0.05).

**[Table 3]**

| Group | Blood GIP level (AUC up to 60 minutes, relative value based on 100 for control group). |
|---|---|
| Control group | 100±5.96 |
| Test group 1 (addition in 0.15 µg per g body weight) | 76.21±10.21 |
| Test group 2 (addition in 2 µg per g body weight) | 72.83±7.24* |

As shown in Table 3, the blood GIP level tends to be lowered in Test group 1, and the blood GIP level was significantly lowered in Test group 2, showing that the cycloartenol exhibits GIP-increase inhibitory effect during the lipid stress.

### Test Example 3 (GIP-increase inhibitory action of cycloartenol during carbohydrate stress)

### (1) Preparation of sample and test method

To white rice (Koshihikari produced in Tochigi prefecture), water (1.5 times equivalent to the volume of the white rice) was added to allow the rice to absorb the water for 30 minutes. Then, the rice was cooked with a rice cooker (ZOJIRUSHI microcomputer-controlled rice cooker NS-KG05, manufactured by Zojirushi Corporation). Water and triolein (manufactured by Sigma-Aldrich Corporation) were added to the cooked rice, followed by homogenization until the mixture was sufficiently homogenized, thereby yielding a sample for administration. The sample for administration was orally probe-administered at 2 mg of white rice per g body weight + 1.5 µg of triolein per g body weight (as a control group). Each of the purified cycloartenol produced in the above-mentioned Example 1 (3) was added to a sample for administration (dissolved in triolein). Each of the resultants was orally probe-administered at 2 mg of white rice per g body weight and 1.5 µg of triolein per g body weight, and 0.075 µg or 0.15 µg of cycloartenol equivalent per g body weight (as Test group 3 or Test group 4).

8-week old male Wistar rats (supplied by Japan SLC, Inc.), after 15 hours of fasting, were divided into groups each consisting of 10 mice, with their total body weight being almost the same between the groups. Immediately after the administration to each group, blood sample was collected from tail vein sequentially. The blood sample was subjected to centrifugation to yield blood plasma, and GIP level in the blood plasma was measured using a GIP measuring kit (manufactured by Linko Research Inc.), and the area under the curve (AUC) in the graph was calculated.

### (2) Test results

Table 4 shows the results of the test. The results are represented as average ± standard error, and statistical significant differences between groups were determined by the Dunnett's test with respect to the control group (*p<0.05).

**[Table 4]**

| Group | Blood GIP level (AUC up to 240 minutes, relative value based on 100 for control group) |
|---|---|
| Control group | 100±5.91 |
| Test group 3 (addition of 0.075 µg per g body weight) | 92.11±10.57 |
| Test group 4 (addition of 0.15 µg per g body weight) | 73.75±9.58* |

As shown in Table 4, the blood GIP level tends to be lowered in Test group 3, and the blood GIP level was significantly lowered in Test group 4, showing that the cycloartenol exhibits GIP-increase inhibitory effect during carbohydrate stress.

## Claims

1. A non-therapeutic in vivo use of a rice bran extract for inhibiting Gastric Inhibitory Polypeptide-increase (GIP-increase).

2. The use according to claim 1, wherein the rice bran extract is a rice oil.

3. The use according to claim 1 or 2, wherein the rice bran extract comprises triterpenes or derivatives thereof.

4. The use according to claim 3, wherein the triterpenes or derivatives thereof are cycloartenol or derivatives thereof.

5. The use according to any one of claims 1 to 4, which involves orally ingesting at 50 µg to 500 mg per day in terms of cycloartenol.

## Patentansprüche

1. Eine nicht-therapeutische in vivo Verwendung eines Reiskleie-Extrakts zur Hemmung der gastrisch inhibitorischen Polypeptid-Zunahme (GIP-Zunahme).

2. Die Verwendung gemäß Anspruch 1, wobei der Reiskleie-Extrakt ein Reisöl ist.

3. Die Verwendung gemäß Anspruch 1 oder 2, wobei der Reiskleie-Extrakt Triterpene oder Derivate davon umfasst.

4. Die Verwendung gemäß Anspruch 3, wobei die Triterpene oder Derivate davon Cycloartenol oder Derivate davon sind.

5. Die Verwendung gemäß einem der Ansprüche 1 bis 4, die die orale Einnahme von 50 µg bis 500 mg pro Tag in Bezug auf Cycloartenol beinhaltet.

## Revendications

1. Utilisation non-thérapeutique *in vivo* d'un extrait de son de riz pour inhiber l'augmentation de polypeptide inhibiteur gastrique (augmentation de GIP).

2. Utilisation selon la revendication 1, dans laquelle l'extrait de son de riz est une huile de riz.

3. Utilisation selon la revendication 1 ou 2, dans laquelle l'extrait de son de riz comprend des triterpènes ou leurs dérivés.

4. Utilisation selon la revendication 3, dans laquelle les triterpènes ou leurs dérivés sont le cycloarténol ou ses dérivés.

5. Utilisation selon l'une quelconque des revendications 1 à 4, qui implique l'ingestion orale de 50 µg à 500 mg par jour de cycloarténol.
